# EUROPÄISCHE PATENTSCHRIFT

(11) **EP 2 903 735 B1**
(45) Veröffentlichungstag und Bekanntmachung des Hinweises auf die Patenterteilung: **14.12.2016**
(21) Anmeldenummer: 13773193.1
(22) Anmeldetag: 24.09.2013
(51) Int. Cl.: B01J 31/04, C07C 51/41, C07C 55/07

(54) **VERFAHREN ZUR HERSTELLUNG VON EDELMETALLOXALAT-KOMPLEXEN**
METHOD FOR PRODUCING PRECIOUS METAL OXALATE COMPLEXES
PROCÉDÉ DE FABRICATION DE COMPLEXES MÉTAL NOBLE-OXALATE

(30) Priorität: 05.10.2012 DE 102012019560; 05.10.2012 US 201261710226 P
(43) Veröffentlichungstag der Anmeldung: 12.08.2015
(73) Patentinhaber: Heraeus Deutschland GmbH & Co. KG, 63450 Hanau (DE)
(72) Erfinder: WALTER, Richard, 63755 Alzenau (DE); EWEINER, Florian, 63452 Hanau (DE); LÄSSIG, Walter, 63571 Gelnhausen (DE); FUCHS ALAMEDA, Jörg, 63599 Biebergemünd (DE)
(74) Vertreter: Heraeus IP
(86) Internationale Anmeldenummer: PCT/EP2013/069787
(87) Internationale Veröffentlichungsnummer: WO 2014/053351

(56) Entgegenhaltungen:
- DATABASE REAXYS [Online] Elsevier; 1902, Kohlschuetter: "Rx-ID: 26372364", XP002718260, Database accession no. Rx-ID: 26372364 & V KOHLSCHUETTER: "Ueber Doppelsalze des Cadmiurns und Quecksilbers", BER. DTSCH. CHEM. GES., Bd. 35, 1. Januar 1902 (1902-01-01), Seiten 483-492, XP055093239,
- DATABASE REAXYS [Online] Elsevier; 1857, Souchay: "Rx-ID: 26372364, 26553716", XP002718261, Database accession no. Rx-ID: 26372364, 26553716 & A Souchay: "Ueher die Oxalate der schweren Metalloxyde", Justus Liebigs Annalen der Chemie, Bd. 102, 1. Januar 1857 (1857-01-01), Seiten 41-54, XP055093241,
- DATABASE REAXYS [Online] Elsevier; 1951, Bisikalova: "RX-ID: 26591514", XP002718262, Database accession no. RX-ID: 26591514 & BISIKALOVA: UKR. KHIM. ZH., Bd. 17, 1951, Seiten 807-814,
- KLAUS KROGMANN ET AL: "Über die "Isomerie" der Dioxalatoplatinate 2. Die freien Säuren", CHEMISCHE BERICHTE, Bd. 99, Nr. 11, 1. November 1966 (1966-11-01), Seiten 3408-3418, XP055093247, ISSN: 0009-2940, DOI: 10.1002/cber.19660991104 in der Anmeldung erwähnt
- Barbara J Keller ET AL: "Solution equilibria leading to the formation of metal-metal bonds in partially oxidized bisoxalatoplatinate(II) systems", INORGANICA CHIMICA ACTA, vol. 357, no. 3, 1 February 2004 (2004-02-01), pages 853-858, XP055265291, NL ISSN: 0020-1693, DOI: 10.1016/j.ica.2003.09.009

## Beschreibung

Die vorliegende Erfindung betrifft die Herstellung von Platinoxalat-Komplexen. Platinoxalat-Komplexe werden im Folgenden der Einfachheit halber auch als Platinoxalate bezeichnet. Sie betrifft insbesondere die Herstellung von Platinoxalat-Komplexen aus Platin-Präkursoren und Oxalsäure und/oder Oxalsäuresalzen.

Die Herstellung von Edelmetalloxalat-Komplexen aus Edelmetall-Präkursoren und Oxalsäure und/oder Oxalsäuresalzen ist seit langem bekannt. Die Herstellung von Platinoxalat-Komplexen erfolgt üblicherweise durch Reaktion von Platinoxid-Hydrat (Platin-(IV)-hydroxosäure, Dihydrogenhexahydroxoplatinat(IV), Hydroxoplatinic acid) mit Oxalsäure bei einer Temperatur von 60°C (K. Krogmann, P. Dodel, Chem. Ber. 1966, 99, 3408-3418).

EP 0 254 935 A1 beschreibt ein Verfahren zur Herstellung von Silberoxalat mit großem Partikeldurchmesser. Nach diesem Verfahren werden Silbersalz und Oxalsäure oder Oxalsäuresalze bei einem pH-Wert von nicht mehr als 5 zur Reaktion gebracht. Die Reaktion wird bei einer Temperatur von 0 bis 80°C, bevorzugt bei einer Temperatur von 40 bis 60°C durchgeführt.

Barbara J. Keller ET AL berichten in "Solution equilibria leading to the formation of metal-metal bonds in partially oxidized bisoxalatoplatinate(II) systems", INORGANICA CHIMICAACTA, Bd. 357, (2004), Seiten 853-858 über die Reaktion von [Pt^{II}(oxalat)₂]²⁻ und geeigneten Oxidationsmitteln unter Bildung des dimeren unverbrückten Platinkomplexes [{Pt^{II}(oxalat)₂}₂]²⁻ sowie die Bildung von Spezies der allgemeinen Formel [{Pt(oxalat)₂}ₙ]ⁿ⁻.

Die Herstellung von Edelmetalloxalat-Komplexen aus Edelmetall-Präkursoren und Oxalsäure und/oder Oxalsäuresalzen ist eine exotherme Reaktion, bei der Wärme und CO₂ gebildet werden. Dabei kann die Temperatur über den Zersetzungspunkt der Edelmetalloxalat-Komplexe ansteigen, wodurch gleichzeitig weiteres CO₂ freigesetzt wird. In diesem Zusammenhang siehe z.B. Sano, Isamu, Bulletin, 15 (1940), 196 ff. "On the Catalytic Decomposition of Oxalic Acid by Colloidal Platinum", und Szabö, Z.G. und Biro-Sugar, E., Zeitschrift für Elektrochemie Bd. 50, Nr. 8, 1956, S. 869-874, "Kinetik der thermischen Zersetzung von Silberoxalat".

Aus sicherheitstechnischen Gründen ist bei der Durchführung der Reaktion in großtechnischem Maßstab daher zu berücksichtigen, dass es nicht zur Zersetzung des Produktes aufgrund von Wärmeentwicklung während der Reaktion kommt.

Aufgabe der vorliegenden Erfindung ist es daher, ein Verfahren zur Herstellung von Platinoxalat-Komplexen bereitzustellen, welches im großtechnischen Maßstab durchgeführt werden kann.

Somit soll ein Verfahren entwickelt werden, das eine kontrollierte Reaktionsführung ermöglicht. Es ist sicherzustellen, dass die bei der Synthese anfallenden Gas- und Wärmemengen vom Reaktor sicher abgeführt werden können.

Diese Aufgabe wird durch ein Verfahren mit den Merkmalen des Anspruchs 1 gelöst. Weiterbildungen der Erfindung sind durch die Unteransprüche definiert.

Erfindungsgemäß wird ein Verfahren zur Herstellung von Platinoxalat-Komplexen bereitgestellt, bei dem Platin-Präkursoren mit Oxalsäure und/oder Oxalsäuresalzen zur Reaktion gebracht werden und bei dem Platinoxalat als Autokatalysator in die Reaktionsmischung eingebracht wird.

Als Edukte werden erfindungsgemäß Platin-Präkursoren und Oxalsäure und/oder Oxalsäuresalze verwendet. Denkbar sind viele Ausgangsstoffe, wobei sich die Edukte Platin-Präkursor und Oxalsäure und/oder Oxalsäuresalze natürlich vom Endprodukt Platinoxalat unterscheiden.

Bei dem Platin-Präkursor handelt es sich um Platinoxid-Hydrat, welches auch als Platin-(IV)-hydroxosäure bezeichnet wird (siehe Gmelin, Verlag Chemie GmbH, Berlin 1940, S. 47-48), oder eines ihrer Salze. Als Ausgangsstoff für Platinoxalat wird Platin-(IV)-hydroxosäure oder eines ihrer Salze, wie z.B. K₂Pt(OH)₆, Na₂Pt(OH)₆ etc., verwendet. Grundsätzlich ist die freie Säure bevorzugt.

Oxalsäuresalze können zum Beispiel Natriumoxalat, Ammoniumoxalat oder Kaliumoxalat oder Mischungen aus diesen sein. Es ist aber auch möglich eine Mischung aus Oxalsäure und einem oder mehreren Oxalsäuresalzen einzusetzen. Grundsätzlich ist jedoch der Einsatz der freien Oxalsäure besonders bevorzugt. So wird auch für die Herstellung von Platinoxalat besonders bevorzugt Oxalsäure verwendet.

Erfindungsgemäß ist eine Edukt-Kombination aus Platinoxid-Hydrat und Oxalsäure besonders bevorzugt, da neben Platinoxalat-Komplexen lediglich Kohlendioxid und Wasser entsteht. Besonders bevorzugt wird Oxalsäure oder Oxalsäuresalz in einem geeigneten stöchiometrischen Verhältnis zugegeben. Im Fall der Herstellung von Platinoxalat-Komplexen bedeutet dies, dass Oxalsäure oder Oxalsäuresalz in 1,8 bis 2,8 molaren Äquivalenten in Bezug auf Platin in Form des Platin-Präkursors zugegeben wird. Bei dieser Reaktion entsteht eine Mischung aus verschiedenen Dioxalatoplatinsäuren oder Platinoxalat-Komplexen. Eine ausführliche Beschreibung solcher Mischungen findet sich in K.Krogmann, P.Dodel, Chem. Ber. 1966, 99, 3402-3407 und 3408-3418.

In welcher Form Oxalsäure und/oder Oxalsäuresalz zugegeben wird, hängt von dem herzustellenden Platinoxalat-Komplex ab. Bevorzugt wird es in Form einer wässrigen Lösung oder in fester Form zugegeben. Oxalsäure ist bevorzugt und wird bevorzugt in fester Form als Oxalsäuredihydrat eingesetzt.

Die Reaktion wird bei einer Temperatur durchgeführt, die unterhalb der Zersetzungstemperatur der Platinoxalat-Komplexe liegt. Für die Festlegung der Sicherheitsspanne bei der Reaktionstemperatur im konkreten Fall ist eine Gefährdungsbeurteilung heranzuziehen, die wichtige verfahrenstechnische Parameter, apparatetechnische Parameter, sicherheitstechnische Überlegungen sowie sicherheitstechnische Daten, wie z.B. die Zersetzungstemperatur bzw. den Zersetzungsbereich der Platinoxalat-Komplexe mit einbezieht. In Abhängigkeit von der Datenlage kann die Reaktionstemperatur an die Zersetzungstemperatur angenähert werden.

Die Reaktion wird somit bevorzugt bei einer Temperatur unterhalb der Zersetzungstemperatur der Platinoxalat-Komplexe durchgeführt. Dabei sollte die Differenz zwischen Reaktionstemperatur und Zersetzungstemperatur mindestens 1°C betragen, bevorzugt ist die Differenz mindestens 5°C. Die Zersetzungstemperatur ist als Temperatur der beginnenden Zersetzung definiert, wobei die beginnende Zersetzung mittels Langzeitdifferenzthermoanalyse in Glasampullen mit einer Heizgeschwindigkeit von 0,05 K/min gemäß Temperatur-Differenz-Thermoanalyse nach DIN 51007 bestimmt wird. Als bevorzugt hat sich für die Reaktion zwischen Platin-Präkursoren und Oxalsäure und/oder Oxalsäuresalzen ein Temperaturbereich zwischen 0°C und 56°C herausgestellt, besonders bevorzugt zwischen 30°C und 52°C und ganz besonders bevorzugt zwischen 35°C und 45°C.

Insbesondere für Platinoxalat-Komplexe, die sich bereits bei einer Temperatur von 57°C zersetzen (siehe Figur), gilt, dass die Reaktion bevorzugt bei einer Temperatur von bis zu 56°C, besonders bevorzugt bis 52°C, und ganz besonders bevorzugt bis 45°C durchgeführt wird. Die Reaktion wird oberhalb von 0°C, bevorzugt oberhalb von 30°C und ganz besonders bevorzugt bei einer Temperatur von 35°C bis 42°C durchgeführt.

Die Zersetzungstemperatur der Platinoxalat-Komplexe ist im Sinne der Erfindung mittels Langzeit-Differenzthermoanalyse (DTA) gemäß DIN51007(Jun1994) zu bestimmen. Die Bestimmung kann an Lösungen von Platinoxalat-Komplexen, welche der Produktlösung entsprechen, in einer geschlossenen Glasampulle mit einer Heizgeschwindigkeit von 0,05 K /min zwischen 0°C und einer Temperatur oberhalb des gemessenen Peakminimums erfolgen (siehe Figur). Als Zersetzungstemperatur wird erfindungsgemäß die Temperatur der ersten Abweichung (siehe Figur, 57°C) der Messkurve von der Anfangsbasiskurve verstanden (5.2 DIN 51007).

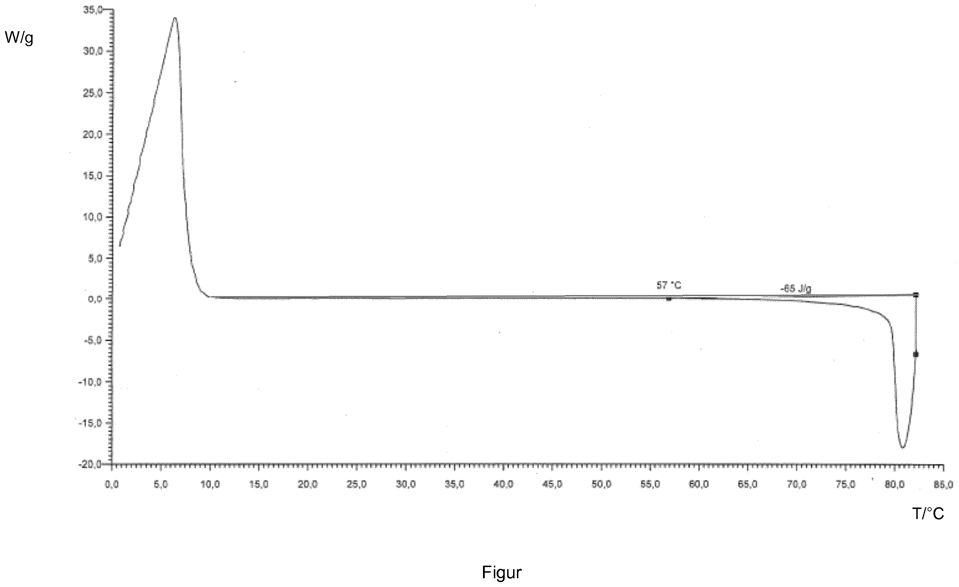

Im konkreten Fall wurden 2934,5 mg einer 10 Gew.-%igen Platinoxalatlösung in Wasser verwendet. Die Messung erfolgte in Glasampullen mit einer Heizgeschwindigkeit von 0,05 K/min. Die Figur zeigt den Wärmefluss W/g in Abhängigkeit von der Temperatur zwischen 2°C und 83°C.

Temperieren bedeutet in vorliegender Beschreibung, dass die Reaktionsmischung auf eine bestimmte Temperatur eingestellt wird. Das Temperieren kann z.B. mit Hilfe von Wasser erfolgen.

Vorteilhaft wird zunächst eine wässrige Lösung oder Suspension aus Platinoxid-Hydrat oder Platinsalz hergestellt. Im Falle der Herstellung von Platinoxalat-Komplexen wird bevorzugt zunächst eine wässrige Suspension aus Platinoxid-Hydrat (H₂[Pt(OH)₆] oder Platin-(IV)-hydroxosäure) hergestellt. Bevorzugt wird eine 5 bis 25 Gew.-%ige Suspension, besonders bevorzugt eine 7-15 Gew.-%ige Suspension bezogen auf Platin in Wasser hergestellt.

Überraschend hat sich gezeigt, dass das Einbringen geringer Mengen von Platinoxalat-Komplexen in die Reaktionsmischung autokatalytisch wirkt. Durch die Zugabe von Platinoxalat-Komplexen wird eine Induktionsperiode der Reaktion (sehr langsame Anfangsphase der Reaktion) signifikant verkürzt. Dadurch wird eine kontrollierte Reaktionsführung ermöglicht. Im Folgenden werden daher die zugefügten Platinoxalat-Komplexe als Autokatalysator bezeichnet. Erfindungsgemäß wird eine geringe Menge Autokatalysator zugegeben. Bevorzugt wird der Autokatalysator in einer Menge von 1×10⁻⁴ bis 5×10⁻² molaren Äquivalenten Platin in Bezug auf das Platin im Platin-Präkursor zugegeben. Besonders bevorzugt wird der Autokatalysator in einer Menge von 5×10⁻⁴ bis 1×10⁻² molaren Äquivalenten Platin in Bezug auf das Platin im Platin-Präkursor zugegeben und ganz besonders bevorzugt wird der Autokatalysator in einer Menge von 5×10⁻⁴ bis 7×10⁻³ molaren Äquivalenten Platin in Bezug auf das Platin im Platin-Präkursor zugegeben. Der Autokatalysator wird bevorzugt in wässriger Lösung zugegeben. Übliche Konzentrationen sind 5-20 Gew.-%, z.B. 8-15 Gew.-%.

Zweckmäßig werden als Autokatalysator (entsprechend der Bedeutung des Begriffs "Autokatalysator") die dem herzustellenden Produkt entsprechenden Platinoxalat-Komplexe eingesetzt. D.h. zur Herstellung von Platinoxalat wird als Autokatalysator Platinoxalat eingesetzt.

Die Reihenfolge der Zuführung von Platin-Präkursor, Autokatalysator und Oxalsäure und/oder Oxalsäuresalz ist von untergeordneter Bedeutung. Der Autokatalysator kann gleichzeitig mit der Gesamtmenge an Oxalsäure und/oder Oxalsäuresalz, mit einem Teil von Oxalsäure und/oder Oxalsäuresalz oder vor der Zugabe von Oxalsäure und/oder Oxalsäuresalz zur Reaktionslösung oder -suspension gegeben werden. Dabei kann entweder eine Lösung oder Suspension aus Platin-Präkursor vorgelegt werden oder später zugegeben werden.

Wird der Platin-Präkursor vorgelegt, und der Autokatalysator gleichzeitig mit Oxalsäure zugegeben, so sollte die Zugabe bei einer Temperatur unterhalb der gewünschten Reaktionstemperatur erfolgen. Bevorzugt erfolgt die Zugabe bei einer Temperatur von bis zu 37°C, besonders bevorzugt bis zu 32°C. Die sich bildende Reaktionsmischung wird anschließend auf die gewünschte Reaktionstemperatur aufgeheizt. Die Geschwindigkeit des Aufheizens richtet sich dann nach dem Einsetzen der Reaktion.

Es hat sich als jedoch als vorteilhaft erwiesen, zunächst den Platin-Präkursor in Lösung oder Suspension vorzulegen, den Autokatalysator zuzugeben und zumindest den größeren Teil der Oxalsäure oder des Oxalsäuresalzes erst nach Erreichen der Reaktionstemperatur zuzugeben.

Die Oxalsäure oder das Oxalsäuresalz kann in einer oder mehreren Portionen zugegeben werden. Die Portionen können gleich groß sein, es können jedoch auch mehrere unterschiedlich große Portionen zugegeben werden. In dem Fall, dass die Portionen nicht gleich groß sind, wird vorteilhaft zunächst eine größere Portion zugegeben und anschließend eine oder mehrere kleinere oder immer kleiner werdende. So hat es sich als vorteilhaft erwiesen, zunächst eine Portion von 0,4 bis 1,4 molaren Äquivalenten bezogen auf Platin in Form des Platin-Präkursors zuzugeben und anschließend z.B. in mehreren gleichen Mengen die restliche Oxalsäure oder das restliche Oxalsäuresalz. Das kann beispielsweise in einer einzigen weiteren Zugabe von z.B. 0,4 bis 1,4 molaren Äquivalenten, in 2 weiteren Zugaben von z.B. 0,2 bis 0,9 molaren Äquivalenten, in 3 weiteren Zugaben von z.B. 0,1 bis 0,7 molaren Äquivalenten, in 4 weiteren Zugaben von z.B. 0,1 bis 0,6 molaren Äquivalenten, usw. erfolgen. Denkbar ist jedoch auch eine gleichmäßige, kontinuierliche Zugabe der Oxalsäure oder des Oxalsäuresalzes.

Vorteilhaft wird die Lösung oder Suspension während der Reaktion gerührt. In der bevorzugten Ausführungsform erfolgt die Zugabe der Oxalsäure oder des Oxalsäuresalzes in Abhängigkeit von den Rührbedingungen, der Konzentration der Lösung oder Suspension und der Reaktordimension. Grundsätzlich lässt sich die Geschwindigkeit, mit der Oxalsäure oder Oxalsäuresalz zugegeben werden kann, gut anhand der CO₂-Entwicklung und der Temperaturentwicklung einstellen.

Auf diese Weise hergestellte Platinoxalat-Komplexe können vorteilhaft als Präkursoren für Platinkatalysatoren eingesetzt werden.

### Beispiele

Die folgenden Beispiele dienen der Erläuterung und sind nicht als Einschränkung zu verstehen.

### Messmethoden, Analysen

Die qualitativen Analysen erfolgten mittels NMR und UV-Spektroskopie.

Das UV-Spektrum wurde bei Zimmertemperatur unter Verwendung eines UV-Spektrometers Specord® 200 der Firma Analytic Jena AG in 1 cm Küvetten (Quarzglasküvetten QS Suprasil® der Firma Heraeus Quarzglas GmbH) in einem Messbereich von 190 nm -1100 nm mit einer Auflösung von 2 nm gemessen.

Die Kernresonanzspektroskopischen Messungen wurden an einem Bruker Avance 400 MHz-NMR-Spektrometer (Vergleichsbeispiel 1) und einem Bruker Avance 600 MHz-NMR Spektrometer (Beispiel, Vergleichsbeispiele 2 und 3) vorgenommen.

Der Platingehalt wurde gravimetrisch bestimmt.

Als Edukte wurden Platin(IV)-hydroxosäure (H₂[Pt(OH)₆]) aus eigener Produktion (w(Pt): 55,51 %), Oxalsäure-Dihydrat zur Analyse EMSURE® ACS, ISO, Reag. Ph Eur der Firma Merck KGaA, Art Nr. 100495 und Platinoxalat aus eigener Produktion (w(Pt): 11,72 %) verwendet.

### Beispiel 1 (Herstellung von Platinoxalat bei 40°C, mit Autokatalysator, Oxalsäurezugabe in 5 Portionen)

In einem 250 ml-Dreihals-Rundkolben wurden 10 g Pt (50 mmol) in Form von 18,01 g H₂[Pt(OH)₆] in 54,29 ml vollentsalztem Wasser (VEW) vorgelegt.

Anschließend wurden bei Zimmertemperatur (23°C) unter Rühren (250 U/min) mittels eines Magnetrührers 0,04 g Pt-Oxalat (0,24 mmol Pt) als Autokatalysator zugegeben. Es entstand eine blass-grünliche Suspension.

### Zeitpunkt: 0 Min:

Die Suspension wurde ausgehend von Zimmertemperatur in einem Wasserbad innerhalb von 20 Minuten auf 40°C aufgeheizt.

### Zeitpunkt 20 Min:

Sobald die Suspension eine Temperatur von 40°C erreicht hatte, wurde eine von fünf gleichen Portionen von je 2,568 g (20 mmol) Oxalsäure-Dihydrat zugegeben. Dabei wurde sofort eine Gasentwicklung beobachtet, die über einen Zeitraum von 60 min andauerte. Es wurden 270 ml CO₂ aufgefangen.

### Zeitpunkt 80 Min:

Nach Abschluss der Gasbildung wurde eine weitere Portion von 2,568 g (20 mmol) Oxalsäure-Dihydrat zugegeben. Es konnten 40 ml CO₂ aufgefangen werden.

### Zeitpunkt 140 Min:

Nach Abschluss der Gasbildung wurde eine weitere Portion von 2,568 g (20 mmol) Oxalsäure-Dihydrat zugegeben. Nach 10 Min. wechselte die Farbe der Lösung von grün ins Türkis-blaue. Innerhalb von 60 Min wurden 300 ml CO₂ aufgefangen.

### Zeitpunkt 200 Min:

Nach Abschluss der Gasbildung wurde eine weitere Portion von 2,568 g (20 mmol) Oxalsäure-Dihydrat zugegeben. Innerhalb von 60 Min wurden 270 ml CO₂ aufgefangen.

### Zeitpunkt 260 Min:

Nach Abschluss der Gasbildung wurde eine weitere Portion von 2,568 g (20 mmol) Oxalsäure-Dihydrat zugegeben. Innerhalb von 110 min konnten 300 ml CO₂ aufgefangen werden. Während weiterer 10 Minuten Rühren bei 40°C wurde keine Gasentwicklung mehr beobachtet.

### Zeitpunkt 380 Min:

Die Heizung wurde abgeschaltet. Die Lösung wurde weitergerührt, bis Zimmertemperatur erreicht wurde.

Die Mischung wurde über einen 0,2 µm Membranfilter (Sartorius Filtrationseinheit) filtriert. Die Filtration erfolgte innerhalb von 30 Minuten.

Es wurden 74,49 g Produkt mit einem Pt-Gehalt von 13,40 Gew.-% in einer Ausbeute von 99,82 % bezogen auf Platin erhalten.
¹³C-NMR (151 MHz, 299,6 K, DMSO-d₆ Kapillare): δ = 168,70; 167,16 ppm UV-VIS: 627 nm (A=0,399); 417 nm (0,415)

### Vergleichsbeispiel 1 (Herstellung von Platinoxalat bei 50°C)

In einem 250 ml-Dreihals-Rundkolben wurden 10 g Pt (50 mmol) in Form von 18,01 g H₂[Pt(OH)₆] in 54,29 ml vollentsalztem Wasser (VEW) vorgelegt. Anschließend wurden unter Rühren (250 U/min) mittels eines Magnetrührers 12,93 g (100 mmol) Oxalsäure-Dihydrat zugegeben. Es entstand eine milchige, gelblich-weiße Suspension.

### Zeitpunkt: 0 Min:

Die Suspension wurde ausgehend von 19°C in einem Wasserbad mit etwa 1°C/10 min erwärmt.

### Zeitpunkt 180 Min:

Bei einer Temperatur von 35°C begann sich die Lösung grünlich zu verfärben.

### Zeitpunkt 210 Min:

Bei einer Temperatur von 38°C wurde die Lösung türkis-blau.

### Zeitpunkt 220 Min:

Bei einer Temperatur von 39°C wurde die Lösung tiefblau.

### Zeitpunkt 230 Min:

Die Lösung erreichte eine Temperatur von 40°C. Es konnte eine Gasentwicklung über einen Zeitraum von 50 Min beobachtet werden, in dem die Lösung eine Temperatur von 45°C erreichte.

### Zeitpunkt 350 Min:

Eine Temperatur von 50°C wurde erreicht. Es fand keine Gasentwicklung mehr statt.

### Zeitpunkt 510 Min:

Die Heizung wurde abgeschaltet, die Lösung wurde weitergerührt, bis Zimmertemperatur erreicht wurde.

Die Mischung wurde über einen 0,2 µm Membranfilter (Sartorius Filtrationseinheit) filtriert. Die Filtration erfolgte innerhalb von 90 Minuten.

Es wurden 47,82 g Produkt mit einem Pt-Gehalt von 20,75 Gew.-% in einer Ausbeute von 99,23 % bezogen auf Platin erhalten.
¹³C-NMR (100,6 MHz, 303 K, DMSO-d₆ Kapillare): δ = 168,43; 166,72 ppm UV-VIS 664 nm (A=0,731); 417 nm (0,763)

### Vergleichsbeispiel 2 (Herstellung von Platinoxalat bei 40°C, ohne Autokatalysator)

Es wurde Vergleichsversuch 1 wiederholt, mit dem Unterschied, dass die Lösung über einen Zeitraum von 210 Minuten von 23°C auf eine Temperatur von 40°C aufgeheizt wurde. Nach 150 Minuten färbte sich die Lösung bei einer Temperatur von 35°C grünlich. Nach 190 Minuten, und bei einer Temperatur von 37°C begann sich die Lösung bläulich zu verfärben und nach 230 Minuten konnte über einen Zeitraum von 65 Minuten eine Gasentwicklung beobachtet werden. Es wurden 80,972 g Produkt mit einem Pt-Gehalt von 12,25 Gew.-% in einer Ausbeute von 99,19 % bezogen auf Platin erhalten.
¹³C-NMR (151 MHz, 298 K, DMSO-d₆ Kapillare): δ = 168,16; 166,67 ppm UV-VIS 641,05 nm (A=0,342); 417 nm (0,374)

### Vergleichsbeispiel 3 (Herstellung von Platinoxalat bei 40°C, ohne Autokatalysator, Oxalsäurezugabe in 5 Portionen)

In einem 250 ml-Dreihals-Rundkolben wurden 10 g Pt (50 mmol) in Form von 18,01 g H₂[Pt(OH)₆] in 54,29 ml vollentsalztem Wasser (VEW) vorgelegt.

### Zeitpunkt 0 Min:

Die Suspension wurde ausgehend von 20°C in einem Wasserbad innerhalb von 40 Minuten auf 40°C aufgeheizt.

### Zeitpunkt 40 Min:

Sobald die Suspension eine Temperatur von 40°C erreicht hatte, wurde eine von fünf gleichen Portionen von je 2,568 g (20 mmol) Oxalsäure-Dihydrat zugegeben. Es wurde weder eine Farbveränderung noch eine Gasentwicklung beobachtet. Nach weiteren 60 Minuten wurde eine weitere Portion von 2,568 g (20 mmol) Oxalsäure-Dihydrat zugegeben. Auch jetzt wurde weder Farbveränderung noch Gasentwicklung beobachtet.

### Zeitpunkt 160 Min:

Eine weitere Portion von 2,568 g (20 mmol) Oxalsäure-Dihydrat wurde zugegeben. 10 Minuten später verfärbte sich die Lösung grünlich. Nach weiteren 30 Min (bei 200 Minuten) wechselte die Farbe der Lösung von grün ins Türkis-blaue.

### Zeitpunkt 220 Min:

Eine weitere Portion von 2,568 g (20 mmol) Oxalsäure-Dihydrat wurde zugegeben. 10 Minuten später konnte eine Gasentwicklung beobachtet werden.

### Zeitpunkt 280 Min:

Es wurde eine weitere Portion von 2,568 g (20 mmol) Oxalsäure-Dihydrat zugegeben. Die Gasentwicklung setzte sich fort bis zum Zeitpunkt 300 Minuten. Danach konnte keine Gasentwicklung mehr beobachtet werden.

### Zeitpunkt 330 Min:

Die Heizung wurde abgeschaltet. Die Lösung wurde weitergerührt, bis Zimmertemperatur erreicht wurde.

Die Mischung wurde über einen 0,2 µm Membranfilter (Sartorius Filtrationseinheit) filtriert. Die Filtration erfolgte innerhalb von 30 Minuten.

Es wurden 77,39 g Produkt mit einem Pt-Gehalt von 12,85 Gew.-% in einer Ausbeute von 99,45 % bezogen auf Platin erhalten.
¹³C-NMR (151 MHz, 299,6 K, DMSO-d₆ Kapillare): δ = 168,16; 166,66 ppm UV-VIS 664 nm (A=0,373); 417 nm (0,403)

## Patentansprüche

1. Verfahren zur Herstellung von Platinoxalat-Komplexen, wobei ein Platin-Präkursor mit Oxalsäure und/oder Oxalsäuresalz zur Reaktion gebracht wird,
**dadurch gekennzeichnet, dass** es sich bei dem Platin-Präkursor um Platinoxid-Hydrat (Platin-(IV)-hydroxosäure) oder eines ihrer Salze handelt und dass dem Produkt entsprechende Platinoxalat-Komplexe als Autokatalysator zugegeben werden.

2. Verfahren zur Herstellung von Platinoxalat-Komplexen nach Anspruch 1,
**dadurch gekennzeichnet,**
**dass** die Reaktion bei einer Temperatur unterhalb der Zersetzungstemperatur der Platinoxalat-Komplexe durchgeführt wird.

3. Verfahren zur Herstellung von Platinoxalat-Komplexen nach Anspruch 2,
**dadurch gekennzeichnet,**
**dass** die Reaktion bei einer maximalen Temperatur von 1°C unterhalb der Zersetzungstemperatur der Platinoxalat-Komplexe durchgeführt wird.

4. Verfahren zur Herstellung von Platinoxalat-Komplexen nach Anspruch 1,
**dadurch gekennzeichnet,**
**dass** die Reaktion bei einer Temperatur zwischen 0 und 56°C durchgeführt wird.

5. Verfahren nach einem der vorhergehenden Ansprüche,
**dadurch gekennzeichnet,**
**dass** im ersten Schritt eine wässrige Lösung oder Suspension aus Platin-Präkursor und der Platinoxalat-Komplexe hergestellt wird,
im zweiten Schritt die wässrige Lösung oder Suspension aus dem ersten Schritt auf Reaktionstemperatur temperiert wird,
und
im dritten Schritt Oxalsäure und/oder Oxalsäuresalz zugegeben wird.

6. Verfahren nach einem der Ansprüche 1 bis 5,
**dadurch gekennzeichnet,**
**dass** der Autokatalysator in einer Menge von 1×10⁻⁴ bis 5×10⁻² molaren Äquivalenten bezogen auf das Platin in der Platin-Präkursorlösung zugegeben wird.

7. Verfahren nach einem der Ansprüche 1 bis 6,
**dadurch gekennzeichnet,**
**dass** Oxalsäure und/oder Oxalsäuresalz aus der Gruppe ausgewählt sind, die aus Oxalsäure, Natriumoxalat, Ammoniumoxalat und Kaliumoxalat und Mischungen aus diesen besteht.

8. Verfahren nach einem der vorhergehenden Ansprüche,
**dadurch gekennzeichnet,**
**dass** die Reaktion bei einer Temperatur von 30°C bis 45°C durchgeführt wird.

9. Verfahren nach einem der vorhergehenden Ansprüche,
**dadurch gekennzeichnet,**
**dass** Oxalsäure in einer Menge von 1,8 bis 2,8 molaren Äquivalenten in Bezug auf Platin im Platin-Präkursor zugegeben wird.

10. Verfahren nach einem der vorhergehenden Ansprüche,
**dadurch gekennzeichnet,**
**dass**
1) eine Suspension von Platinoxid-Hydrat (Platin-(IV)-hydroxosäure) in Wasser hergestellt wird,
2) eine Lösung von Platinoxalat-Komplexen in Wasser hergestellt wird,
3) die Suspension aus 1) und die Lösung aus 2) vereinigt werden und die resultierende Mischung auf die Reaktionstemperatur temperiert wird,
4) eine erste Portion von 0,4 bis 1,4 molaren Äquivalenten Oxalsäure bezogen auf Platin in Platinoxid-Hydrat (Platin-(IV)-hydroxosäure) zugegeben wird, und
5) eine zweite Portion von 0,1 bis 1,4 molaren Äquivalenten Oxalsäure bezogen auf Platin in Platinoxid-Hydrat (Platin-(IV)-hydroxosäure) zugegeben wird, und
6) optional Schritt 5) 1 bis mehrmals wiederholt wird, bis eine Gesamtmenge von 1,8 bis 2,8 molaren Äquivalenten Oxalsäure in Bezug auf Platin in Platinoxid-Hydrat (Platin-(IV)-hydroxosäure) zugegeben worden ist.

## Claims

1. Method for the production of platinum oxalate complexes, whereby a platinum precursor is reacted with oxalic acid and/or oxalic acid salt,
**characterised in that** the platinum precursor is a platinum oxide-hydrate (platinum-(IV)-hydroxo acid) or a salt thereof and **in that** corresponding platinum oxalate complexes are added to the product as auto-catalysts.

2. Method for the production of platinum oxalate complexes according to claim 1, **characterised in that**
the reaction is made to take place at a temperature below the decomposition temperature of the platinum oxalate complexes.

3. Method for the production of platinum oxalate complexes according to claim 2, **characterised in that**
the reaction is made to take place at a maximal temperature of 1°C below the decomposition temperature of the platinum oxalate complexes.

4. Method for the production of platinum oxalate complexes according to claim 3, **characterised in that**
the reaction is made to take place at a temperature between 0 and 56°C.

5. Method according to any one of the preceding claims,
**characterised in that**
an aqueous solution or suspension is produced from platinum precursor and the platinum oxalate complexes in the first step,
the aqueous solution or suspension from the first step is heated to reaction temperature in the second step,
and
oxalic acid and/or oxalic acid salt is/are added in the third step.

6. Method according to any one of the claims 1 to 5,
**characterised in that**
the amount of the auto-catalyst added is 1 x 10⁻⁴ to 5 x 10⁻² molar equivalents relative to the platinum in the platinum precursor solution.

7. Method according to any one of the claims 1 to 6,
**characterised in that**
oxalic acid and/or oxalic acid salt are selected from the group consisting of oxalic acid, sodium oxalate, ammonium oxalate and potassium oxalate and mixtures thereof.

8. Method according to any one of the preceding claims,
**characterised in that**
the reaction is made to take place at a temperature of 30°C to 45°C.

9. Method according to any one of the preceding claims,
**characterised in that**
the amount of oxalic acid added is 1.8 to 2.8 molar equivalents relative to platinum in the platinum precursor.

10. Method according to any one of the preceding claims,
**characterised in that**
1) a suspension of platinum oxide-hydrate (platinum-(IV)-hydroxo acid) in water is produced;
2) a solution of platinum oxalate complexes in water is produced;
3) the suspension from 1) and the solution from 2) are combined and the resulting mixture is heated to the reaction temperature;
4) a first aliquot of 0.4 to 1.4 molar equivalents of oxalic acid, relative to platinum in platinum oxide-hydrate (platinum-(IV)-hydroxo acid), is added; and
5) a second portion of 0.1 to 1.4 molar equivalents of oxalic acid, relative to platinum in platinum oxide-hydrate (platinum-(IV)-hydroxo acid), is added;
and
6) optionally, step 5) is repeated once or multiple times until a total amount of 1.8 to 2.8 molar equivalents of oxalic acid, relative to platinum in platinum oxide-hydrate (platinum-(IV)-hydroxo acid), has been added.

## Revendications

1. Procédé de fabrication de complexes d'oxalate de platine, dans lequel un précurseur de platine est mis à réagir avec de l'acide oxalique et/ou un sel d'acide oxalique,
**caractérisé en ce qu'**il s'agit pour le précurseur de platine d'hydrate d'oxyde de platine (acide platine-(IV)-hydroxylique) ou d'un de ses sels et que des complexes d'oxalate de platine correspondants sont ajoutés au produit en tant qu'autocatalyseur.

2. Procédé de fabrication de complexes d'oxalate de platine selon la revendication 1,
**caractérisé en ce**
**que** la réaction est réalisée à une température en dessous de la température de décomposition des complexes d'oxalate de platine.

3. Procédé de fabrication de complexes d'oxalate de platine selon la revendication 2,
**caractérisé en ce**
**que** la réaction est réalisée à une température maximale de 1°C en dessous de la température de décomposition des complexes d'oxalate de platine.

4. Procédé de fabrication de complexes d'oxalate de platine selon la revendication 1,
**caractérisé en ce**
**que** la réaction est réalisée à une température comprise entre 0 et 56°C.

5. Procédé selon une des revendications précédentes,
**caractérisé en ce**
**qu'**une solution ou suspension aqueuse est fabriquée à partir d'un précurseur de platine et des complexes d'oxalate de platine dans la première étape, la solution ou suspension aqueuse provenant de la première étape est tempérée à la température réactionnelle dans la deuxième étape,
et
de l'acide oxalique et/ou un sel d'acide oxalique est ajouté dans la troisième étape.

6. Procédé selon une des revendications 1 à 5,
**caractérisé en ce**
**que** l'autocatalyseur est ajouté en une quantité de 1×10⁻⁴ à 5×10⁻² équivalents molaires par rapport au platine dans la solution de précurseur de platine.

7. Procédé selon une des revendications 1 à 6,
**caractérisé en ce**
**que** l'acide oxalique et/ou le sel d'acide oxalique sont sélectionnés parmi le groupe qui se compose de l'acide oxalique, l'oxalate de sodium, l'oxalate d'ammonium et l'oxalate de potassium et des mélanges de ceux-ci.

8. Procédé selon une des revendications précédentes,
**caractérisé en ce**
**que** la réaction est réalisée à une température de 30°C à 45°C.

9. Procédé selon une des revendications précédentes,
**caractérisé en ce**
**que** l'acide oxalique est ajouté en une quantité de 1,8 à 2,8 équivalents molaires par rapport au platine dans le précurseur de platine.

10. Procédé selon une des revendications précédentes,
**caractérisé en ce**
**que**
1) une suspension d'hydrate d'oxyde de platine (acide platine-(IV)-hydroxylique) dans de l'eau est fabriquée,
2) une solution de complexes d'oxalate de platine dans de l'eau est fabriquée,
3) la suspension de 1) et la solution de 2) sont réunies et le mélange résultant est tempéré à la température réactionnelle,
4) une première portion de 0,4 à 1,4 équivalent molaire d'acide oxalique par rapport au platine dans l'hydrate d'oxyde de platine (acide platine-(IV)-hydroxylique) est ajoutée, et
5) une seconde portion de 0,1 à 1,4 équivalent molaire d'acide oxalique par rapport au platine dans l'hydrate d'oxyde de platine (acide platine-(IV)-hydroxylique) est ajoutée, et
6) en option l'étape 5) est répétée 1 à plusieurs fois jusqu'à ce qu'une quantité totale de 1,8 à 2,8 équivalents molaires d'acide oxalique par rapport au platine dans l'hydrate d'oxyde de platine (acide platine-(IV)-hydroxylique) ait été ajoutée.
